(19) Europäisches Patentamt
European Patent Office
Office européen des brevets

(11) EP 4 620 423 A1

(12) **EUROPEAN PATENT APPLICATION**
published in accordance with Art. 153(4) EPC

(43) Date of publication:
24.09.2025 Bulletin 2025/39

(21) Application number: 24757144.1

(22) Date of filing: 08.02.2024

(51) International Patent Classification (IPC):
A61B 34/30 (2016.01)        A61B 34/37 (2016.01)
B25J 9/16 (2006.01)          A61B 90/00 (2016.01)
A61B 17/00 (2006.01)

(52) Cooperative Patent Classification (CPC):
A61B 17/00; A61B 34/30; A61B 34/37;
A61B 90/00; B25J 9/16

(86) International application number:
PCT/KR2024/001928

(87) International publication number:
WO 2024/172415 (22.08.2024 Gazette 2024/34)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 14.02.2023 KR 20230019061

(71) Applicant: LN Robotics Inc.
Seoul 05505 (KR)

(72) Inventors:
• WON, Jong Seok
Seoul 06226 (KR)
• CHOI, Ju Eun
Seoul 04906 (KR)
• PARK, Sang Eun
Seoul 05285 (KR)

(74) Representative: Lavoix
Bayerstraße 83
80335 München (DE)

(54) **SURGICAL TOOL CONTROL SYSTEM**

(57) A surgical tool control system according to an embodiment comprises: a driving assembly comprising a first roller module and a second roller module disposed in parallel such that a surgical tool is retained therebetween; a manipulation assembly for receiving a command input from a user to be transferred to the driving assembly; and a processor for controlling operations of the driving assembly according to a selected mode. If a first mode is selected, the processor may rotate the first and second roller modules such that the surgical tool is moved forward/backward in response to the user's command that has been input, or may move at least one of the first and second roller modules vertically such that the surgical tool is rotated. If a second mode is selected, the processor may reciprocally move at least one of the first and second roller modules vertically such that the surgical tool is reciprocally rotated by a designated reciprocally rotating angle.

FIG. 4

**Description**

**TECHNICAL FIELD**

The following embodiments relates to a surgical tool control system.

**BACKGROUND ART**

In the conventional percutaneous coronary intervention (PCI) procedure, an operator is continuously exposed to radiation, and considerable time and cost are required to train skilled practitioners to a level that allows stable procedures to be performed. Furthermore, variations in procedural quality among operators, regions, and medical institutions make it challenging to ensure the consistent delivery of high-quality medical services. To address these issues, robotic assistance systems for interventional procedures have been introduced. For example, an interventional procedure assistive robot may be configured to advance, retract, or rotate a surgical tool in response to commands input by a user.

The above description is information the inventor(s) acquired during the course of conceiving the present disclosure, or already possessed at the time, and was not necessarily publicly known before the present application was filed.

Korean Laid-open Patent Publication No. 10-2019-0121928 (published on October 29, 2019) discloses a driving device for a medical robot and a medical robot.

**DISCLOSURE OF THE INVENTION**

**TECHNICAL GOALS**

An aspect of an embodiment provides a surgical tool control system with improved operability.

Another aspect of an embodiment provides a surgical tool control system that facilitates entry into a lesion site.

**TECHNICAL SOLUTIONS**

According to an embodiment, a surgical tool control system includes a driving assembly including a first roller module and a second roller module arranged in parallel such that a surgical tool is gripped between the first roller module and the second roller module; a control assembly configured to receive a command from a user to be transmitted to the driving assembly; and a processor that may control an operation of the driving assembly according to a selected mode, wherein when a first mode is selected, the processor may rotate the first roller module and the second roller module to cause the surgical tool to move forward or backward in response to the user input or move at least one of the first roller module and the second roller module in a vertical direction such that the surgical tool rotates, and when a second mode is selected, the processor may reciprocate at least one of the first roller module and the second roller module in a vertical direction such that the surgical tool rotates back and forth by a designated reciprocating rotation angle.

When the second mode is selected, the processor may move at least one of the first roller module and the second roller module back and forth in a vertical direction by a circumferential length of the surgical tool corresponding to the reciprocating rotation angle.

To calculate the circumferential length of the surgical tool corresponding to the reciprocating rotation angle, a diameter of the surgical tool may be either pre-stored in the processor or measured by a sensor.

To calculate the circumferential length of the surgical tool corresponding to the reciprocating rotation angle, the diameter of the surgical tool is measured based on a distance between the first roller module and the second roller module while the surgical tool is gripped between the first roller module and the second roller module.

When the second mode is selected and a command to move the surgical tool forward or backward is input to the control assembly, the processor may rotate the first roller module and the second roller module such that the surgical tool moves forward or backward while rotating back and forth in response to the user command.

When the second mode is selected, the control assembly may receive a command for at least one of the reciprocating rotation angle, a speed at which the surgical tool rotates back and forth, and a speed at which the surgical tool moves forward or backward.

The control assembly may include a control handle that is movable forward or backward along a first axis and rotatable around the first axis.

When the second mode is selected and the control handle is rotated in either direction around the first axis, the processor may increase or decrease the reciprocating rotation angle.

When the second mode is selected and the control handle is rotated in either direction around the first axis, the processor may increase or decrease the speed at which the surgical tool rotates back and forth.

When the second mode is selected and the control handle moves forward or backward along the first axis, the processor may change the speed at which the surgical tool moves forward or backward.

The control assembly may include a jog wheel that is rotatable in discrete steps by a first angle about a second axis.

When the second mode is selected, every time the jog wheel rotates by the first angle in either direction about the second axis, the processor may increase or decrease the reciprocating rotation angle in discrete steps by a second angle.

When the second mode is selected, every time the jog wheel rotates by the first angle in either direction about the second axis, the processor may increase or decrease the speed at which the surgical tool rotates back and forth in discrete steps by a first magnitude.

When the second mode is selected and a pause command is input by the user, the processor may stop the vertical movement of the first roller module and the second roller module to stop the rotation of the surgical tool.

When the second mode is selected, every time the jog wheel rotates by the first angle in either direction about the second axis, the processor may generate a signal that causes the surgical tool to move forward or backward by a first pitch.

When the second mode is selected, the processor may change the speed at which the surgical tool rotates back and forth based on a predetermined pattern.

## EFFECTS OF THE INVENTION

A surgical tool control system according to an embodiment may improve the operability for the user.

The surgical tool control system according to an embodiment may facilitate access to a lesion site.

The effects of the surgical tool control system according to an embodiment are not limited to the above-mentioned effects, and other effects not mentioned herein will be clearly understood from the following description by those of ordinary skill in the art.

## BRIEF DESCRIPTION OF DRAWINGS

FIG. 1 is a schematic perspective view of a surgical tool control system according to an embodiment.
FIG. 2 is a perspective view of a driving assembly according to an embodiment.
FIG. 3 is a plan view of the driving assembly according to an embodiment.
FIG. 4 illustrates a state in which the driving assembly according to an embodiment is used.
FIG. 5 illustrates a process in which a roller module of the driving assembly according to an embodiment moves a surgical tool forward or backward.
FIG. 6 illustrates a process in which a roller module of the driving assembly according to an embodiment rotates a surgical tool.
FIG. 7 is a perspective view of a control assembly according to an embodiment.
FIG. 8 is a schematic diagram illustrating a situation in which a first roller module and a second roller module according to an embodiment rotate a first surgical tool back and forth.
FIG. 9 is a schematic perspective view illustrating a situation in which a surgical tool enters an example lesion site.
FIG. 10 is a schematic perspective view illustrating a situation in which a surgical tool enters an example vascular branch.

## BEST MODE FOR CARRYING OUT THE INVENTION

Hereinafter, embodiments will be described in detail with reference to the accompanying drawings. Various modifications may be made to the embodiments. Here, the embodiments are not construed as limited to the disclosure and should be understood to include all changes, equivalents, and replacements within the idea and the technical scope of the disclosure.

The terminology used herein is for the purpose of describing particular embodiments only and is not to be limiting of the embodiments. As used herein, the singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined herein, all terms used herein including technical or scientific terms have the same meanings as those generally understood by one of ordinary skill in the art. Terms, such as those defined in commonly used dictionaries, should be interpreted as having a meaning that is consistent with their contextual usage in the relevant technical field and will not be interpreted in an idealized or overly formal sense unless expressly so defined herein.

When describing the embodiments with reference to the accompanying drawings, like reference numerals refer to like components and a repeated description related thereto will be omitted. In the following description, well-known functions or constructions are not described in detail since they would obscure the invention in unnecessary detail.

In the following description of embodiments, terms such as first, second, A, B, (a), and (b) may be used to describe various components, but such terms are merely used to distinguish one component from another and do not imply a specific order or essence of the components. When a component is referred to as being "connected," "coupled," or "joined" to another component, it can be directly connected, coupled, or joined to the other component, or intervening components may be present.

Elements having the same or similar functions as those described in one embodiment are designated by the same reference numerals in other embodiments. Unless otherwise stated, descriptions made in any one embodiment are applicable to other embodiments, and redundant explanations are omitted for the sake of brevity.

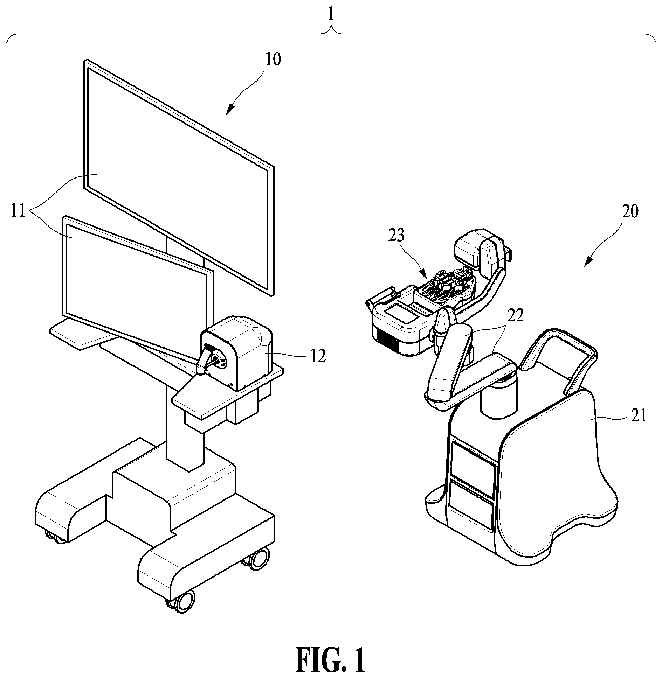

FIG. 1 is a schematic perspective view of a surgical tool control system according to an embodiment.

Referring to FIG. 1, a surgical tool control system 1 according to an embodiment may receive a control command input by a user and drive the surgical tool based on the input command. For example, the surgical tool control system 1 may be used for vascular interventional procedures. However, this is merely an example, and the use of the surgical tool control system 1 is not limited thereto.

According to an embodiment, the surgical tool control system 1 may include a master part 10 and a slave part 20. The master part 10 may receive a command from the user and display information. The slave part 20 may receive a command from the master part 10 and drive a surgical tool based on the received command. For example, the slave part 20 may be positioned in a procedure space provided with an X-ray device, and the master part 10 may be positioned in a shielded space separated from the procedure space. Accordingly, since the user may input the command through the master part 10 in the shielded space, the user may not be exposed to radiation generated during X-ray imaging.

According to an embodiment, the master part 10 may include a display 11, a control assembly 12, and a processor (not shown). The display 11 may provide visual information to the user. For example, the display 11 may present information related to the slave part 20 or display X-ray images. The display 11 may receive an input from the user via a touch panel. The control assembly 12 may receive a command from the user to operate a surgical tool. The command input through the control assembly 12 may be transmitted to the processor, and the processor may control a driving assembly 23 based on the input command.

According to an embodiment, the processor (not shown) may control the driving assembly 23 according to a selected mode. For example, the processor may include a CPU and/or memory. The processor may be provided within master part 10; however, this is merely an example, and a portion or all of the processor may be provided in the slave part 20. The user may select a surgical tool control mode through the display 11 and/or the control assembly 12. For example, the surgical tool control mode may include a first mode and a second mode. A detailed description of the first mode and the second mode will be provided below.

According to an embodiment, the slave part 20 may include a slave base 21, an arm 22, and the driving assembly 23. The slave base 21 may provide a base where the slave part 20 is installed. The driving assembly 23 may be connected to the slave base 21 via at least one arm 22. For example, the at least one arm 22 may connect the driving assembly 23 to the slave base 21 through a link structure. The position of the driving assembly 23 relative to the slave base 21 may be adjustable via the at least one arm 22. At least one surgical tool may be mounted to the driving assembly 23. The driving assembly 23 may drive the at least one mounted surgical tool. For example, the driving assembly 23 may advance, retract, or rotate the surgical tool. The driving assembly 23 may receive a command from the master part 10 and operate the at least one surgical tool based on the received command.

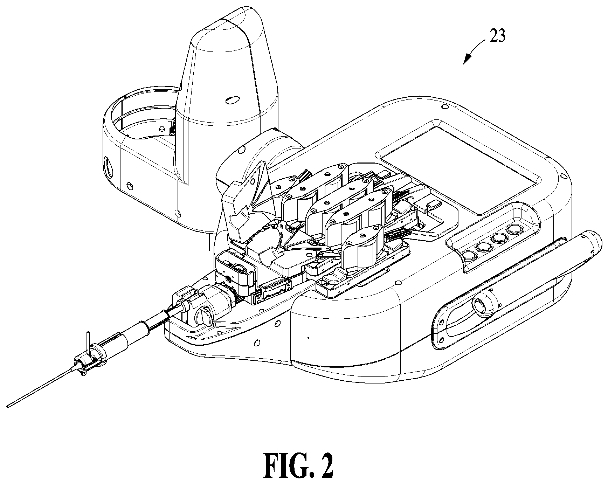

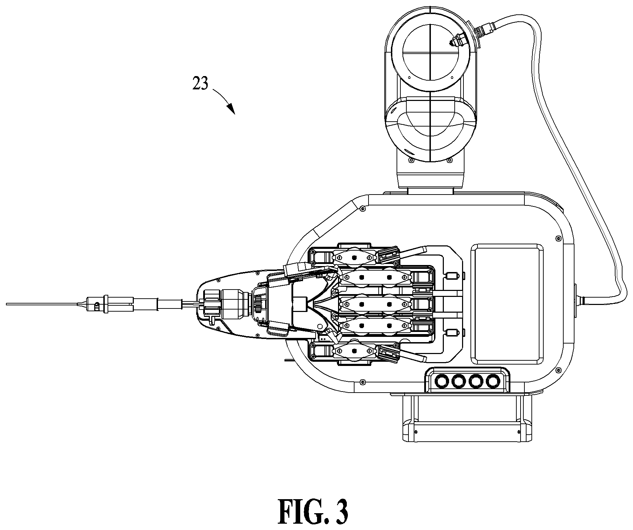

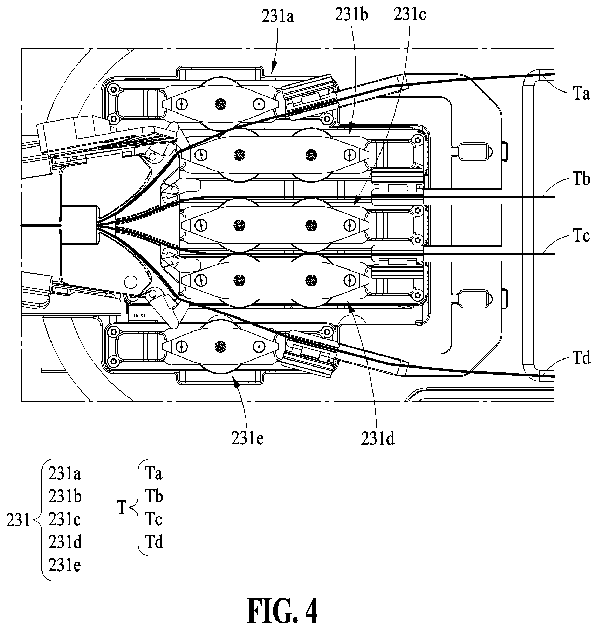

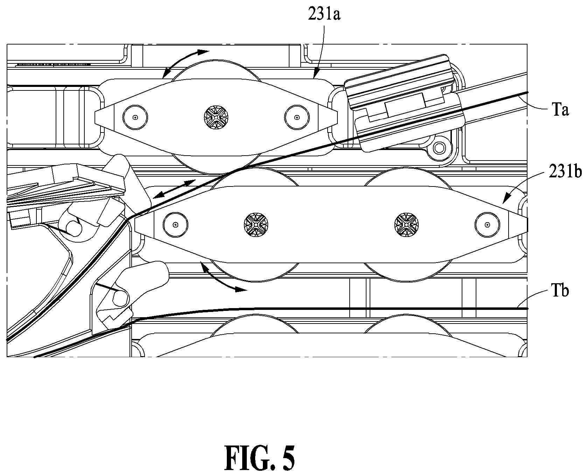

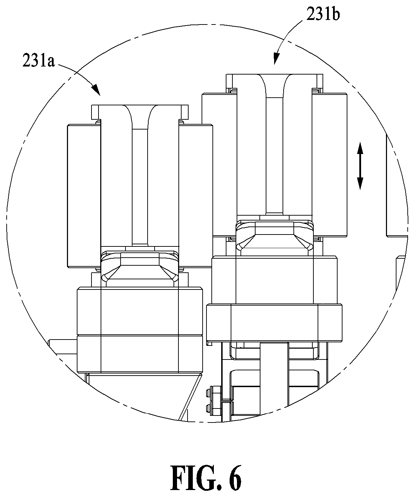

FIG. 2 is a perspective view of a driving assembly according to an embodiment. FIG. 3 is a plan view of the driving assembly according to an embodiment. FIG. 4 illustrates a state in which the driving assembly according to an embodiment is used. FIG. 5 illustrates a process in which a roller module of the driving assembly according to an embodiment moves a surgical tool forward or backward. FIG. 6 illustrates a process in which a roller module of the driving assembly according to an embodiment rotates a surgical tool.

Referring to FIGS. 2 to 6, the driving assembly 23 may independently control a plurality of surgical tools T. Here, the surgical tool T may refer to a tool having a longitudinal direction. For example, the surgical tool T may include various types of tools such as guidewires or balloon catheters that have a longitudinal direction. However, this is merely an example, and the type of the surgical tool T is not limited thereto.

According to an embodiment, the driving assembly 23 may include a plurality of roller modules 231. The roller modules 231 may be provided as at least a pair. For example, as illustrated in the drawings, the roller modules 231 may include a first roller module 231a, a second roller module 231b, a third roller module 231c, a fourth roller module 231d, and a fifth roller module 231e. However, this is merely an example, and the number of roller modules 231 is not limited thereto. The plurality of roller modules 231 may be arranged in parallel with one another.

According to an embodiment, a surgical tool T may be gripped between two adjacent roller modules 231. To this end, at least one of the roller modules 231 may be translatable in a horizontal direction toward another roller module 231. For example, as illustrated in FIG. 4, the second roller module 231b may translate toward the first roller module 231a such that a first surgical tool Ta is gripped between the first roller module 231a and the second roller module 231b. In this case, a second surgical tool Tb positioned between the second roller module 231b and the third roller module 231c may be released from gripping. Conversely, the second roller module 231b may translate toward the third roller module 231c such that the second surgical tool Tb is gripped between the second roller module 231b and the third roller module 231c. In this case, the first surgical tool Ta positioned between the first roller module 231a and the second roller module 231b may be released from gripping.

Likewise, the fourth roller module 231d may translate in a horizontal direction toward the third roller module 231c such that a third surgical tool Tc is gripped between the third roller module 231c and the fourth roller module 231d. In this case, a fourth surgical tool Td positioned between the fourth roller module 231d and the fifth roller module 231e may be released from gripping. Conversely, the fourth roller module 231d may translate toward the fifth roller module 231e such that a fourth surgical tool Td is gripped between the fourth roller module 231d and the fifth roller module 231e. In this case, the third surgical tool Tc positioned between the third roller module 231c and the fourth roller module 231d may be released from gripping.

According to an embodiment, the driving assembly 23 may implement the advancement, retraction, and rotation of the surgical tool T through rotation and vertical movement of the roller modules 231. Specifically, as illustrated in FIG. 5, in a state where the first roller module 231a and the second roller module 231b are positioned adjacent to each other and grip the first surgical tool Ta therebetween, the first roller module 231a and the second roller module 231b may rotate in one direction or the other, thereby moving the gripped first surgical tool Ta forward or backward along its longitudinal direction. In addition, as illustrated in FIG. 6, in the state where the first roller module 231a and the second roller module 231b are positioned adjacent to each other and grip the first surgical tool Ta therebetween, at least one of the roller modules 231a and 231b may move in a vertical direction, thereby rotating the gripped first surgical tool Ta.

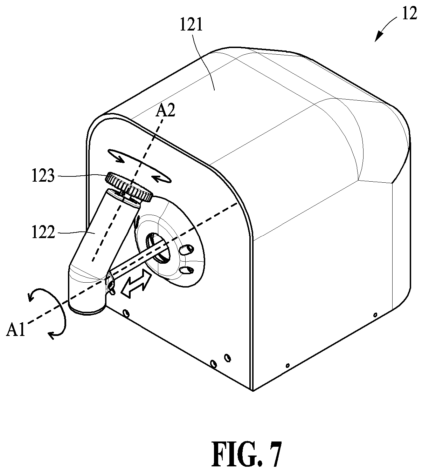

FIG. 7 is a perspective view of a control assembly according to an embodiment.

Referring to FIGS. 1 and 7, the control assembly 12 according to an embodiment may receive a command to be transmitted to the driving assembly 23. The command input through the control assembly 12 may be delivered to the processor, and the processor may control the driving assembly 23 based on the input command. The control assembly 12 may include a base housing 121, a control handle 122, and a jog wheel 123.

According to an embodiment, the base housing 121 may form at least a portion of the exterior of the control assembly 12. The base housing 121 may be fixedly installed in the master part 10. The base housing 121 may provide an internal space in which various components may be disposed. For example, components such as a shaft, a gear, a motor, a wire, and/or a pulley may be disposed inside the base housing 121. However, this is merely an example, and the components disposed inside the base housing 121 are not limited thereto.

According to an embodiment, the control handle 122 may receive a command from a user to control the driving assembly 23. The control handle 122 may have a shape that is easy for the user to grip with one hand. Meanwhile, the position, shape, and/or size of the control handle 122 shown in the drawings are merely illustrative and are not limited to those illustrated. The control handle 122 may be connected to the base housing 121 to be movable forward and backward and/or rotatable.

According to an embodiment, the control handle 122 may be movable forward and/or backward relative to the base housing 121 along a first axis A1. The control handle 122 may also be rotatable in one direction and/or the other about the first axis A1 relative to the base housing 121. However, the direction of the first axis A1 illustrated in the drawings is merely exemplary and is not limited thereto.

According to an embodiment, the jog wheel 123 may receive a command from the user to control the driving assembly 23. For example, the jog wheel 123 may be a component used to more precisely control a surgical tool compared to the control handle 122. The jog wheel 123 may be coupled to the control handle 122. The jog wheel 123 may be rotatable in one direction or the other about a second axis A2 relative to the control handle 122. When the user grips the control handle 122, the user's finger (e.g., a thumb or an index finger) may be positioned on the job wheel 123. Meanwhile, the position, shape, and/or size shown in the drawings are illustrative and are not limited to those illustrated. In addition, the direction of the second axis A2 is exemplary and is not limited thereto.

According to an embodiment, the jog wheel 123 may be discretely rotatable about the second axis A2 relative to the control handle 122 by an increment of a first angle. As the jog wheel 123 is configured to discretely rotate in discrete increments of the first angle, the user may rotate the jog wheel 123 in unit steps (i.e., by the first angular increment). For example, the first angle may be 5 degrees. With such a configuration, the jog wheel 123 may have a total of 72 discrete rotational increments. However, this is merely an example, and the first angle is not limited thereto. Meanwhile, two or more jog wheels 123 may be provided.

Hereinafter, a case where a first mode is selected will be described with reference to FIGS. 1 to 7.

According to an embodiment, the first mode may be a default mode and serve as a basic control mode. When the first mode is selected, the processor may rotate the roller modules 231 (e.g., the first roller module 231a and the second roller module 231b) such that the surgical tool T (e.g., the first surgical tool Ta) moves forward or backward, or may move at least one of the roller modules 231 (e.g., the first roller module 231a and the second roller module 231b) in a vertical direction such that the surgical tool T (e.g., the first surgical tool Ta) rotates. For example, in the state where the first mode is selected, the user may freely move the surgical tool T forward, backward, and/or rotate it by inputting a command through the control assembly 12. The first mode may be selected in a general situation in which the surgical tool T is inserted into a blood vessel. However, this is merely an example, and the situations in which the first mode is used are not limited thereto.

According to an embodiment, when the first mode is selected and the control handle 122 moves forward or backward along the first axis A1, the processor may generate a signal for advancing or retracting the surgical tool T. The signal generated by the processor may be transmitted to the driving assembly 23, and the roller modules 231 may rotate to move the surgical tool T, which is gripped by the roller modules 231, forward or backward. With this configuration, in the first mode, when the user moves the control handle 122 forward along the first axis A1, the surgical tool T may be advanced, and when the user moves the control handle 122 backward along the first axis A1, the surgical tool T may be retracted. Depending on the extent to which the control handle 122 moves forward or backward along the first axis A1, the processor may vary the speed at which the surgical tool T is advanced or retracted. For example, the processor may adjust the advancement or retraction speed of the surgical tool T proportionally to the stroke by which the control handle 122 moves along the first axis A1. Alternatively, the processor may discretely adjust the advancement or retraction speed of the surgical tool T based on a section in which the stroke of the control handle 122 falls along the first axis A1. For example, the processor may discretely adjust the speed to 0.5 times, 1 time, or 2 times depending on the section. The manner in which the speed of the surgical tool T is adjusted based on the movement of the control handle 122 may vary depending on user settings.

According to an embodiment, when the first mode is selected and the control handle 122 rotates in one direction or the other around the first axis A1, the processor may generate a signal for rotating the surgical tool T in one direction or the other. The signal generated by the processor may be transmitted to the driving assembly 23, and at least one of the roller modules 231 may move in a vertical direction such that the surgical tool T, gripped by the roller modules 231, rotates in one direction or the other. With this configuration, in the first mode, when the user rotates the control handle 122 clockwise around the first axis A1, the surgical tool T may be rotated in the clockwise direction, and when the user rotates the control handle 122 counterclockwise around the first axis A1, the surgical tool T may be rotated in the counterclockwise direction. Depending on the extent to which the control handle 122 is rotated around the first axis A1, the processor may vary the speed at which the surgical tool T is rotated. For example, the processor may adjust the rotation speed of the surgical tool T in proportion to the rotation angle of the control handle 122. Alternatively, the processor may discretely adjust the rotation speed of the surgical tool T based on a section in which the rotation angle of the control handle 122 falls. For example, when the rotation angle of the control handle 122 falls within a first section, the rotation speed of the surgical tool T may be set to a first speed, and when the rotation angle of the control handle 122 falls within a second section, the rotation speed of the surgical tool T may be set to a second speed. The manner in which the rotation speed of the surgical tool T is adjusted based on the rotation of the control handle 122 may vary depending on user settings.

According to an embodiment, when the first mode is selected, and the control handle 122 is in a state of being moved forward, backward, or rotated, the surgical tool T may continuously advance, retract, or rotate accordingly. When the force applied to the control handle 122 is removed, the control handle 122 may return to its original position. For example, when the user releases the force after moving or rotating the control handle 122, the control handle 122 may return to the original position. When the control handle 122 returns to its original position, the operation of the surgical tool T may be stopped.

According to an embodiment, when the first mode is selected and the jog wheel 123 rotates in one direction or the other around the second axis A2, the processor may generate a signal to rotate the surgical tool T in the corresponding direction. The rotation signal generated by the processor may be transmitted to the driving assembly 23, and at least one of the roller modules 231 may move in a vertical direction such that the surgical tool T gripped between the roller modules 231 is rotated in the corresponding direction. With this configuration, when the user rotates the jog wheel 123 clockwise around the second axis A2, the surgical tool T may be rotated in the clockwise direction, and when the user rotates the jog wheel 123 counterclockwise around the second axis A2, the surgical tool T may be rotated in the counterclockwise direction.

According to an embodiment, when the first mode is selected, the processor may generate a signal to rotate the surgical tool T by a second angle whenever the jog wheel 123 is rotated by a first angle. That is, each time the user rotates the jog wheel 123 by one step (i.e., the first angle), the surgical tool T may be rotated by the second angle. The second angle may be set to be the same as the first angle. For example, when the first angle is 5 degrees, the second angle may also be set to 5 degrees. Alternatively, the second angle may be set as a value obtained by multiplying the first angle by a scale factor. The scale factor may be less than 1. For example, if the first angle is 5 degrees, the second angle may be set to 1 degree. Preferably, the second angle may be set within a range of 1 to 5 degrees. However, this is merely an example, and the second angle is not limited thereto. The second angle may vary depending on user settings.

According to an embodiment, when the first mode is selected, the processor may be configured to generate a signal for advancing or retracting the surgical tool T in response to the rotation of the jog wheel 123. For example, each time the jog wheel 123 rotates in one direction or the other by the first angle, the processor may generate a signal to move the surgical tool T forward or backward by a first pitch. The first pitch, which is the distance by which the surgical tool T is advanced or retracted per one-step rotation of the jog wheel 123, may vary depending on user settings. For example, the first angle may range from 1 to 5 degrees, and the first pitch may range from 0.5 mm to 1.5 mm. In one example, the first angle may be 5 degrees, and the first pitch may be 1 mm. However, these are merely examples, and the first angle and/or the first pitch are not limited thereto. In the first mode, whether the jog wheel 123 causes rotation or advancement/retraction of the surgical tool T may be changed based on user selection.

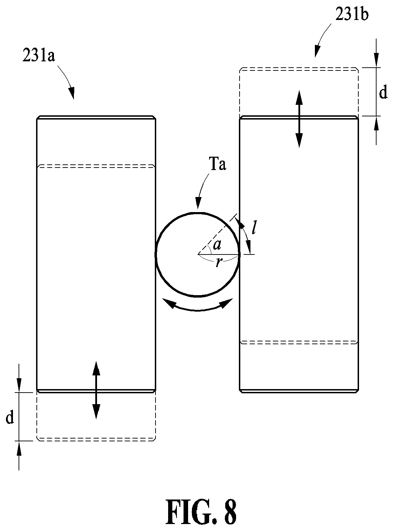

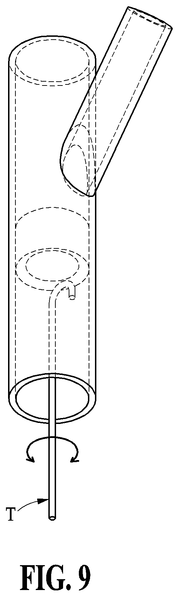

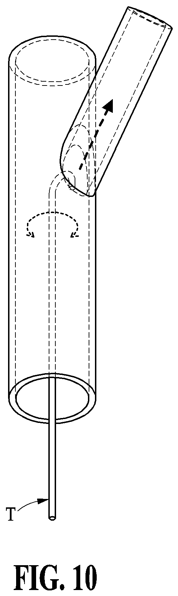

FIG. 8 is a schematic diagram illustrating a situation in which a first roller module and a second roller module according to an embodiment rotate a first surgical tool back and forth. FIG. 9 is a schematic perspective view illustrating a situation in which a surgical tool enters an example lesion site. FIG. 10 is a schematic perspective view illustrating a situation in which a surgical tool enters an example vascular branch.

Hereinafter, a case in which a second mode is selected will be described with reference to FIGS. 1 to 10.

In an embodiment, the second mode may refer to a mode in which the surgical tool T automatically rotates back and forth by a specified reciprocating rotation angle a. For example, in the second mode, the surgical tool T may repeatedly rotate in one direction and then in the opposite direction, each by the reciprocating rotation angle a. Since the surgical tool T automatically rotates by reciprocating in the second mode, this mode may be advantageous in situations where the surgical tool T is required to penetrate an occluded part of a blood vessel. For example, as illustrated in FIG. 9, the second mode may be employed in a situation where the surgical tool T needs to penetrate a lesion site. Similarly, the second mode may also be advantageous in situations where the surgical tool T needs to enter a vascular branch, as illustrated in FIG. 10. However, these are merely examples, and the situations in which the second mode is used are not limited thereto.

In an embodiment, the reciprocating rotation angle a may be selectively set or modified by a user. For example, the reciprocating rotation angle a may be selected within a range greater than 0° and less than 360°. For example, as illustrated in FIG. 9, in a case where the surgical tool needs to pass through a lesion site, the reciprocating rotation angle a may be set to 90° to 180°. As illustrated in FIG. 10, in a case where the surgical tool needs to enter a vascular branch, the reciprocating rotation angle a may be set to 45° to 90°. However, these are merely examples, and the reciprocating rotation angle a is not limited thereto.

In an embodiment, the default speed at which the surgical tool T rotates back and forth in the second mode may be set within a range of 45°/sec to 360°/sec. However, this is merely an example, and the default reciprocating rotation speed of the surgical tool T in the second mode may vary depending on user settings. In addition, the reciprocating rotation speed in the second mode may be modified in real time by the user.

In an embodiment, when the second mode is selected, a processor may reciprocate at least one of the roller modules 231 (e.g., the first roller module 231a and the second roller module 231b) gripping the surgical tool T (e.g., the first surgical tool Ta) in a vertical direction such that the surgical tool T (e.g., the first surgical tool Ta) rotates back and forth by the specified reciprocating rotation angle a. For example, in the second mode, at least one of the roller modules 231 gripping the surgical tool T may repeatedly move up and down. The processor may reciprocate at least one of the roller modules 231 (e.g., the first roller module 231a and the second roller module 231b) in the vertical direction by a circumferential length 1 of the surgical tool (e.g., the first surgical tool Ta) corresponding to the reciprocating rotation angle a. That is, a distance d by which at least one of the roller modules 231 (e.g., the first roller module 231a and the second roller module 231b) gripping the surgical tool T (e.g., the first surgical tool Ta) reciprocates in the vertical direction may be set to the same as the circumferential length 1 of the surgical tool T corresponding to the reciprocating rotation angle a. The circumferential length 1 of the surgical tool T corresponding to the reciprocating rotation angle a may be calculated using a diameter 2r of the surgical tool T. The circumferential length 1 may be calculated as $l = 2\pi r \times \frac{a}{360}$. Accordingly, to rotate the surgical tool T back and forth by the specified reciprocating rotation angle a, the processor may reciprocate at least one of the roller modules 231 in the vertical direction by $2\pi r \times \frac{a}{360}$. For example, as illustrated in FIG. 8, when at least one of the first roller module 231a and the second roller module 231b gripping the first surgical tool Ta repeats upward and downward motion by $2\pi r \times \frac{a}{360}$, the first surgical tool Ta may rotate back and forth by the corresponding reciprocating rotation angle a. Under such control, in the second mode, the surgical tool T may rotate back and forth by the reciprocating rotation angle a. However, this is merely an example, and considering friction between the first surgical tool Ta and the first roller module 231a and the second roller module 231b gripping the first surgical tool Ta, the distance by which at least one of the first roller module 231a and the second roller module 231b moves upward or downward may be set to be greater than the length calculated in the foregoing manner. For example, it may be configured to move by a distance greater than the calculated length by a predetermined value or by a length obtained by multiplying the calculated length by a predetermined factor.

In an embodiment, to calculate the circumferential length l of the surgical tool T corresponding to the reciprocating rotation angle a, the diameter 2r of the surgical tool T may be pre-stored in the processor. For example, the processor may include a database storing the diameters 2r of various types of surgical tools T. The processor may retrieve and match the corresponding diameter 2r based on the type of the mounted surgical tool T from the stored database. The type of the mounted surgical tool T may be input by the user, recognized by a sensor, or set to a predetermined default value.

In an embodiment, to calculate the circumferential length l of the surgical tool T corresponding to the reciprocating rotation angle a, the diameter 2r of the surgical tool T may be measured by the sensor. For example, a separate sensor configured to measure the diameter 2r of the mounted surgical tool T may be provided in the driving assembly 23.

In an embodiment, to calculate the circumferential length l of the surgical tool T corresponding to the reciprocating rotation angle a, the diameter 2r of the surgical tool T may be measured as a distance between the roller modules 231 while the surgical tool T is gripped by the roller modules 231. For example, when the first surgical tool Ta is gripped between the first roller module 231a and the second roller module 231b, the distance between the first roller module 231a and the second roller module 231b may be measured as the diameter 2r of the first surgical tool Ta. Meanwhile, in an embodiment, the diameter 2r or the radius r of the surgical tool T may also be directly input to the processor by the user.

In an embodiment, when the second mode is selected, the control assembly 12 may receive a command for the reciprocating rotation angle a, the speed at which the surgical tool T rotates back and forth, and/or the speed at which the surgical tool T moves forward or backward. That is, in the second mode, the processor may change the reciprocating rotation angle a, modify the speed at which the surgical tool T rotates back and forth, or move the surgical tool T forward or backward while the surgical tool T is rotating back and forth, in response to the input command. Meanwhile, this is merely an example, and the user may input a command for the reciprocating rotation angle a, the speed at which the surgical tool T rotates back and forth, and/or the speed at which the surgical tool T moves forward or backward via a display 1 of the master part 10, in addition to the control assembly 12.

In an embodiment, when the second mode is selected, and a command to move the surgical tool T (e.g., the first surgical tool Ta) forward or backward is input to the control assembly 12, the processor may rotate the roller modules 231 (e.g., the first roller module 231a and the second roller module 231b) such that the surgical tool T (e.g., the first surgical tool Ta) moves forward or backward while rotating back and forth, in response to the user input. The manner in which the control assembly 12 receives the forward or backward command for the surgical tool T while the second mode is selected may be substantially the same as in the case where the first mode is selected.

In an embodiment, when the second mode is selected and the control handle 122 is moved forward or backward along the first axis A1, the processor may generate a signal to advance or retract the surgical tool T. The signal generated by the processor may be transmitted to the driving assembly 23, and the roller modules 231 may be rotated such that the surgical tool T, gripped by the roller modules 231, is moved forward or backward. Depending on the extent to which the control handle 122 is moved along the first axis A1, the processor may vary the speed at which the surgical tool T is advanced or retracted. For example, the processor may proportionally adjust the advancement or retraction speed of the surgical tool T based on the stroke by which the control handle 122 is moved along the first axis A1. Alternatively, the processor may discretely adjust the advancement or retraction speed of the surgical tool T based on a section to which the stroke of the control handle 122 belongs along the first axis A1. For example, the processor may discretely adjust the speed to 0.5 times, 1 time, or 2 times depending on the corresponding section. The manner in which the advancement or retraction speed of the surgical tool T is adjusted based on the movement of the control handle 122 may vary depending on user settings. With such control, the user may advance or retract the surgical tool T while maintaining its reciprocating rotation in the second mode. Accordingly, when the second mode is used in situations such as penetrating through a lesion site, as illustrated in FIG. 9, or entering a vascular branch, as illustrated in FIG. 10, the operability may be improved.

In an embodiment, when the second mode is selected and the control handle 122 is rotated in one direction or the other about the first axis A1, the processor may decrease or increase the reciprocating rotation angle a of the surgical tool T. For example, the processor may adjust the reciprocating rotation angle a of the surgical tool T in proportion to the rotation angle of the control handle 122. Alternatively, the processor may discretely decrease or increase the reciprocating rotation angle a of the surgical tool T according to a section in which the rotation angle of the control handle 122 falls. For instance, when the rotation angle of the control handle 122 falls within a first section, the reciprocating rotation angle a of the surgical tool T may be decreased or increased by a first magnitude, and when the rotation angle falls within a second section, the reciprocating rotation angle a may be decreased or increased by a second magnitude. When the second mode is selected, if the force applied to the control handle 122 disappears (e.g., when the user releases the force after rotating the control handle 122), the control handle 122 may return to its original position, and the reciprocating rotation angle a of the surgical tool T may be restored to a default value. For example, the system may be configured such that the reciprocating rotation angle a of the surgical tool T changes only while the control handle 122 is being rotated. However, this is merely an example, and the manner in which the reciprocating rotation angle a of the surgical tool T is adjusted based on the rotation of the control handle 122 in the second mode may vary according to user settings.

According to an embodiment, when the second mode is selected and the control handle 122 is rotated in one direction or the other about the first axis A1, the processor may increase or decrease the reciprocating rotation speed of the surgical tool T. For example, the processor may adjust the reciprocating rotation speed of the surgical tool T in proportion to the rotation angle of the control handle 122. Alternatively, the processor may discretely increase or decrease the reciprocating rotation speed of the surgical tool T based on a section in which the rotation angle of the control handle 122 falls. For example, when the rotation angle of the control handle 122 falls within a first section, the reciprocating rotation speed of the surgical tool T may be increased or decreased by a first magnitude, and when the rotation angle of the control handle 122 falls within a second section, the reciprocating rotation speed may be increased or decreased by a second magnitude. When the second mode is selected, if the force applied to the control handle 122 disappears (e.g., when the user releases the force after rotating the control handle 122), the control handle 122 may return to its original position, and the reciprocating rotation speed of the surgical tool T may be restored to a default value. For example, the system may be configured such that the reciprocating rotation speed of the surgical tool T is changed only while the control handle 122 is being rotated. However, this is merely an example, and the manner in which the reciprocating rotation speed of the surgical tool T is adjusted based on the rotation of the control handle 122 in the second mode may vary depending on user settings. In addition, whether the rotation of the control handle 122 is used to adjust the reciprocating rotation angle or the reciprocating rotation speed of the surgical tool T in the second mode may be selected by the user.

According to an embodiment, when the second mode is selected and the jog wheel 123 is rotated in one direction or the other about the second axis A2, the processor may increase or decrease the reciprocating rotation angle a. For example, each time the jog wheel 123 is rotated by a first angle, the processor may discretely increase or decrease the reciprocating rotation angle a by a second angle. That is, in the second mode, each time the user rotates the jog wheel 123 by one step (i.e., the first angle), the reciprocating rotation angle a of the surgical tool T may be increased or decreased by the second angle. For example, the second angle may be set within a range of 30° to 90°. In one example, the second angle may be set equal to the first angle. For example, when the first angle is 5 degrees, the second angle may also be set to 5 degrees, equal to the first angle. Alternatively, the second angle may be set to a value obtained by multiplying the first angle by a scale factor. For example, the scale factor may be set to a value greater than or less than 1. However, these are merely examples, and the second angle is not limited thereto. The second angle may vary depending on user settings.

According to an embodiment, when the second mode is selected and the jog wheel 123 is rotated in one direction or the other about the second axis A2, the processor may increase or decrease the reciprocating rotation speed of the surgical tool T. For example, each time the jog wheel 123 is rotated by a first angle, the processor may discretely increase or decrease the reciprocating rotation speed of the surgical tool T by a first magnitude. That is, in the second mode, each time the user rotates the jog wheel 123 by one step (i.e., the first angle), the reciprocating rotation speed of the surgical tool T may be increased or decreased by the first magnitude. For example, the first magnitude may be set within a range of 30°/sec to 90°/sec. However, this is merely an example, and the first magnitude is not limited thereto. The first magnitude may vary depending on user settings.

According to an embodiment, when the second mode is selected, the processor may be configured to generate a signal to advance or retract the surgical tool T in response to the rotation of the jog wheel 123. For example, each time the jog wheel 123 is rotated in one direction or the other by a first angle, the processor may generate a signal to move the surgical tool T forward or backward by a first pitch. The first pitch, which is the distance the surgical tool T is moved forward or backward per one-step rotation of the jog wheel 123, may vary depending on user settings. For example, the first angle may be within a range of 1 to 5 degrees, and the first pitch may be within a range of 0.5 mm to 1.5 mm. In one example, the first angle may be 5 degrees, and the first pitch may be 1 mm. Whether the jog wheel 123 is configured to change the reciprocating rotation angle, the reciprocating rotation speed, or the forward/reverse movement of the surgical tool T in the second mode may vary depending on user selection.

According to an embodiment, when the second mode is selected and a pause command is input by the user, the processor may stop the vertical movement of the roller modules 231 (e.g., the first roller module 231a and the second roller module 231b) gripping the surgical tool T (e.g., the first surgical tool Ta), such that the rotation of the surgical tool T is paused. When a resume command is input by the user while in the paused state, the processor may cause at least one of the roller modules 231 (e.g., the first roller module 231a and the second roller module 231b) to resume vertical reciprocation so that the surgical tool T (e.g., the first surgical tool Ta) is again rotated back and forth automatically.

According to an embodiment, when the second mode is selected, the processor may change the reciprocating rotation speed of the surgical tool T based on a predefined pattern. For example, when the second mode is selected and the user inputs an automatic control command, the speed at which the surgical tool T rotates may be automatically varied according to the predefined pattern. For example, the predefined pattern may be one in which the speed varies in the form of a trigonometric waveform, a triangular waveform, or a pulse waveform. However, these patterns are merely examples and are not limited thereto. The variation pattern of the speed may be set or adjusted in various manners by the user.

According to an embodiment, when the second mode is selected and the jog wheel 123 rotates in one direction or the other about the second axis A2, the processor may increase or decrease the continuous rotation speed of the surgical tool T. For example, each time the jog wheel 123 is rotated by the first angle, the processor may discretely increase or decrease the continuous rotation speed of the surgical tool T by a first magnitude. That is, in the second mode, each time the user rotates the jog wheel 123 by one step (i.e., the first angle), the continuous rotation speed of the surgical tool T may be increased or decreased by the first magnitude. For example, the first magnitude may be set within a range of 360°/sec to 900°/sec. However, this is merely an example, and the first magnitude is not limited thereto. The first magnitude may vary depending on user settings.

According to an embodiment, when the second mode is selected, the processor may be configured to generate a signal to advance or retract the surgical tool T in response to the rotation of the jog wheel 123. For example, each time the jog wheel 123 rotates in one direction or the other by the first angle, the processor may generate a signal to move the surgical tool T forward or backward by a first pitch. The first pitch, which is the distance by which the surgical tool T is advanced or retracted per one-step rotation of the jog wheel 123, may vary depending on user settings. For example, the first angle may range from 1 to 5 degrees, and the first pitch may range from 0.5 mm to 1.5 mm. In one example, the first angle may be 5 degrees, and the first pitch may be 1 mm. Whether the jog wheel 123 changes the continuous rotation speed of the surgical tool T or causes the surgical tool T to be advanced or retracted in the second mode may vary depending on user selection.

According to an embodiment, when the second mode is selected and a pause command is input by the user, the processor may stop the vertical movement of the roller modules 231 (e.g., the first roller module 231a and the second roller module 231b) gripping the surgical tool T (e.g., the first surgical tool Ta) such that the rotation of the surgical tool T is paused. When a resume command is input by the user in the paused state, the processor may cause at least one of the roller modules 231 (e.g., the first roller module 231a and the second roller module 231b) to continuously move in a vertical direction to resume the continuous rotation of the surgical tool T (e.g., the first surgical tool Ta).

According to an embodiment, when the second mode is selected, the processor may change the continuous rotation speed of the surgical tool T based on a predefined pattern. For example, when the second mode is selected and the user inputs an automatic control command, the rotation speed of the surgical tool T may automatically change according to the predefined pattern. For example, the predefined pattern may be a pattern in which the magnitude of the speed changes in a trigonometric waveform, triangular waveform, or pulse waveform. However, the above-described patterns are merely examples, and are not limited thereto. The speed variation pattern may be set or adjusted in various manners by the user.

Although the present disclosure has been described with reference to the limited drawings, those skilled in the art will appreciate that various technical modifications and alterations can be made based on the above. For example, the described technology may be carried out in a different order than described, and/or the components of the described systems, structures, devices, and circuits may be combined or configured in a different form or replaced or substituted by other components or equivalents.

Accordingly, other implementations, other embodiments, and equivalents to the claims also fall within the scope of the following claims.

**Claims**

1.  A surgical tool control system comprising:

    a driving assembly comprising a first roller module and a second roller module arranged in parallel such that a surgical tool is gripped between the first roller module and the second roller module;
    a control assembly configured to receive a command input by a user to be transmitted to the driving assembly; and
    a processor configured to control an operation of the driving assembly according to a selected mode,
    wherein, when a first mode is selected, the processor is configured to, in response to the user input, rotate the first roller module and the second roller module to cause the surgical tool to move forward or backward, or move at least one of the first and second roller modules in a vertical direction such that the surgical tool rotates, and
    when a second mode is selected, the processor is configured to reciprocate at least one of the first roller module and the second roller module in a vertical direction such that the surgical tool rotates back and forth by a specified reciprocating rotation angle.

2.  The surgical tool control system of claim 1, wherein, when the second mode is selected, the processor is configured to move at least one of the first roller module and the second roller module back and forth in a vertical direction by a circumferential length of the surgical tool corresponding to the reciprocating rotation angle.

3.  The surgical tool control system of claim 2, wherein, to calculate the circumferential length of the surgical tool corresponding to the reciprocating rotation angle, a diameter of the surgical tool is either pre-stored in the processor or measured by a sensor.

4.  The surgical tool control system of claim 2, wherein, to calculate the circumferential length of the surgical tool corresponding to the reciprocating rotation angle, the diameter of the surgical tool is measured based on a distance between the first roller module and the second roller module while the surgical tool is gripped between the first roller module and the second roller module.

5. The surgical tool control system of claim 1, wherein, when the second mode is selected and a command to move the surgical tool forward or backward is input to the control assembly, the processor is configured to rotate the first roller module and the second roller module such that the surgical tool moves forward or backward in response to the command input by the user while the surgical tool is rotating back and forth.

6. The surgical tool control system of claim 1, wherein, when the second mode is selected, the control assembly is configured to receive a command for at least one of the reciprocating rotation angle, a speed at which the surgical tool rotates back and forth, and a speed at which the surgical tool moves forward or backward.

7. The surgical tool control system of claim 6, wherein the control assembly comprises a control handle that is movable forward or backward along a first axis and rotatable around the first axis.

8. The surgical tool control system of claim 7, wherein, when the second mode is selected and the control handle is rotated in either direction around the first axis, the processor is configured to decrease or increase the reciprocating rotation angle.

9. The surgical tool control system of claim 7, wherein, when the second mode is selected and the control handle is rotated in either direction about the first axis, the processor is configured to decrease or increase a speed at which the surgical tool rotates back and forth.

10. The surgical tool control system of claim 7, wherein, when the second mode is selected and the control handle moves forward or backward along the first axis, the processor is configured to change the speed at which the surgical tool moves forward or backward.

11. The surgical tool control system of claim 6, wherein the control assembly comprises a jog wheel that is rotatable in discrete steps by a first angle about a second axis.

12. The surgical tool control system of claim 11, wherein, when the second mode is selected, every time the jog wheel rotates by the first angle in either direction about the second axis, the processor is configured to decrease or increase the reciprocating rotation angle in discrete steps by a second angle.

13. The surgical tool control system of claim 11, wherein, when the second mode is selected, every time the jog wheel rotates by the first angle in either direction about the second axis, the processor is configured to decrease or increase the speed at which the surgical tool rotates back and forth in discrete steps by a first magnitude.

14. The surgical tool control system of claim 11, wherein, when the second mode is selected, every time the jog wheel rotates by the first angle in either direction about the second axis, the processor is configured to generate a signal that causes the surgical tool to move forward or backward by a first pitch.

15. The surgical tool control system of claim 1, wherein, when the second mode is selected, the processor is configured to change the speed at which the surgical tool rotates back and forth based on a predetermined pattern.

1

10

11

12

23

20

22

21

FIG. 1

23

FIG. 2

**FIG. 3**

231a  231b  231c

Ta

Tb

Tc

Td

231e  231d

$$231 \begin{cases} 231a \\ 231b \\ 231c \\ 231d \\ 231e \end{cases} \quad T \begin{cases} Ta \\ Tb \\ Tc \\ Td \end{cases}$$

# FIG. 4

FIG. 5

**FIG. 6**

FIG. 7

**FIG. 8**

**FIG. 9**

FIG. 10

**EP 4 620 423 A1**

## INTERNATIONAL SEARCH REPORT

| | |
|---|---|
| International application No. | |
| | **PCT/KR2024/001928** |

**A. CLASSIFICATION OF SUBJECT MATTER**

**A61B 34/30**(2016.01)i; **A61B 34/37**(2016.01)i; **B25J 9/16**(2006.01)i; **A61B 90/00**(2016.01)i; **A61B 17/00**(2006.01)i

According to International Patent Classification (IPC) or to both national classification and IPC

**B. FIELDS SEARCHED**

Minimum documentation searched (classification system followed by classification symbols)

A61B 34/30(2016.01); A61B 17/00(2006.01); A61B 17/28(2006.01); A61B 17/94(2006.01); A61B 19/00(2006.01); A61M 25/09(2006.01); A61M 25/092(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 시술 도구 제어(treatment tool control), 롤러쌍(a pair of rollers), 전후진(forward and backward movement), 회전(rotation), 왕복 회전(alternating rotation), 시술 도구 직경(treatment tool diameter), 조작 핸들(control handle), 조그 휠(jog wheel)

**C. DOCUMENTS CONSIDERED TO BE RELEVANT**

| Category* | Citation of document, with indication, where appropriate, of the relevant passages | Relevant to claim No. |
|---|---|---|
| X | KR 10-2018-0039049 A (ROBOCATH) 17 April 2018 (2018-04-17)<br>See abstract; paragraphs [0001], [0022], [0062] and [0079]-[0109]; claims 2 and 21; and figures 1, 2a, 3, 4c, 5c and 6a. | 1-6,15 |
| Y | | 7-14 |
| Y | KR 10-2010-0077615 A (INDUSTRY FOUNDATION OF CHONNAM NATIONAL UNIVERSITY) 08 July 2010 (2010-07-08)<br>See abstract; paragraphs [0065]-[0068]; and figures 3-6. | 7-14 |
| Y | KR 10-2014-0037108 A (MEDROBOTICS CORPORATION) 26 March 2014 (2014-03-26)<br>See abstract; paragraphs [0215]-[0220]; and figures 1 and 5a. | 7-10 |
| A | KR 10-2022-0068090 A (THE ASAN FOUNDATION et al.) 25 May 2022 (2022-05-25)<br>See entire document. | 1-15 |

☑ Further documents are listed in the continuation of Box C. ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 May 2024** | **17 May 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

22

**INTERNATIONAL SEARCH REPORT**

| International application No. |
| --- |
| **PCT/KR2024/001928** |

| C. | DOCUMENTS CONSIDERED TO BE RELEVANT | |
| --- | --- | --- |
| Category* | Citation of document, with indication, where appropriate, of the relevant passages | Relevant to claim No. |
| A | JP 2017-018619 A (CORINDUS, INC.) 26 January 2017 (2017-01-26)<br>See entire document. | 1-15 |
| A | KR 10-2010-0011477 A (MEERE COMPANY INC.) 03 February 2010 (2010-02-03)<br>See entire document. | 1-15 |

Form PCT/ISA/210 (second sheet) (July 2022)

## INTERNATIONAL SEARCH REPORT
### Information on patent family members

International application No.

**PCT/KR2024/001928**

| Patent document cited in search report | | | Publication date (day/month/year) | Patent family member(s) | | | Publication date (day/month/year) |
|---|---|---|---|---|---|---|---|
| KR | 10-2018-0039049 | A | 17 April 2018 | CN | 107847279 | A | 27 March 2018 |
| | | | | CN | 107847279 | B | 07 January 2022 |
| | | | | EP | 3307194 | A1 | 18 April 2018 |
| | | | | JP | 2018-524136 | A | 30 August 2018 |
| | | | | JP | 6942696 | B2 | 29 September 2021 |
| | | | | KR | 10-2478359 | B1 | 16 December 2022 |
| | | | | US | 10695533 | B2 | 30 June 2020 |
| | | | | US | 2018-0185616 | A1 | 05 July 2018 |
| | | | | WO | 2016-198799 | A1 | 15 December 2016 |
| KR | 10-2010-0077615 | A | 08 July 2010 | KR | 10-1084724 | B1 | 22 November 2011 |
| KR | 10-2014-0037108 | A | 26 March 2014 | CN | 103619271 | A | 05 March 2014 |
| | | | | CN | 103619271 | B | 09 July 2019 |
| | | | | EP | 2629655 | A2 | 28 August 2013 |
| | | | | EP | 2629655 | B1 | 19 December 2018 |
| | | | | JP | 2014-500070 | A | 09 January 2014 |
| | | | | JP | 5711380 | B2 | 30 April 2015 |
| | | | | KR | 10-1786832 | B1 | 18 October 2017 |
| | | | | KR | 10-2014-0027229 | A | 06 March 2014 |
| | | | | US | 2014-0005683 | A1 | 02 January 2014 |
| | | | | US | 8992421 | B2 | 31 March 2015 |
| | | | | WO | 2012-054829 | A2 | 26 April 2012 |
| | | | | WO | 2012-054829 | A3 | 19 July 2012 |
| KR | 10-2022-0068090 | A | 25 May 2022 | CN | 116348172 | A | 27 June 2023 |
| | | | | EP | 4249031 | A1 | 27 September 2023 |
| | | | | JP | 2023-546438 | A | 02 November 2023 |
| | | | | KR | 10-2511036 | B1 | 17 March 2023 |
| | | | | US | 2024-0013907 | A1 | 11 January 2024 |
| | | | | WO | 2022-108102 | A1 | 27 May 2022 |
| JP | 2017-018619 | A | 26 January 2017 | EP | 2282803 | A1 | 16 February 2011 |
| | | | | EP | 2282803 | B1 | 01 July 2015 |
| | | | | JP | 2011-519678 | A | 14 July 2011 |
| | | | | JP | 5723766 | B2 | 27 May 2015 |
| | | | | US | 2010-0069833 | A1 | 18 March 2010 |
| | | | | US | 7887549 | B2 | 15 February 2011 |
| | | | | WO | 2009-137410 | A1 | 12 November 2009 |
| KR | 10-2010-0011477 | A | 03 February 2010 | CN | 102014760 | A | 13 April 2011 |
| | | | | CN | 102014760 | B | 06 November 2013 |
| | | | | KR | 10-0994101 | B1 | 12 November 2010 |
| | | | | KR | 10-1013081 | B1 | 14 February 2011 |
| | | | | KR | 10-2009-0127481 | A | 14 December 2009 |
| | | | | US | 2011-0022229 | A1 | 27 January 2011 |
| | | | | WO | 2009-151206 | A1 | 17 December 2009 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190121928